# EUROPEAN PATENT SPECIFICATION

(11) **EP 3 070 475 B1**
(45) Date of publication and mention of the grant of the patent: **19.02.2020**
(21) Application number: 16161135.5
(22) Date of filing: 18.03.2016
(51) Int. Cl.: G01N 33/68

(54) **IN VITRO DIAGNOSTIC, PROGNOSIS OF CONTRAST INDUCED NEPHROPATHY**
IN-VITRO DIAGNOSTIKUM, PROGNOSE VON KONTRASTMITTEL-INDUZIERTER NEPHROPATHIE
DIAGNOSTIC IN VITRO, PRONOSTIC DE LA NÉPHROPATHIE INDUITE DE CONTRASTE

(30) Priority: 18.03.2015 DE 102015104088
(43) Date of publication of application: 21.09.2016
(73) Proprietor: Kellner, Karl-Heinz, 76131 Karlsruhe (DE)
(72) Inventor: Kellner, Karl-Heinz, 76131 Karlsruhe (DE)
(74) Representative: Benedum, Ulrich Max

(56) References cited:
- HOLMES E W ET AL: "OXIDATIVE TRYPTOPHAN METABOLISM IN RENAL ALLOGRAFT RECIPIENTS: INCREASED KYNURENINE SYNTHESIS IS ASSOCIATED WITH INFLAMMATION AND OKT3 THERAPY", CYTOKINE, ACADEMIC PRESS LTD, PHILADELPHIA, PA, US, vol. 4, no. 3, 1 May 1992 (1992-05-01), pages 205-213, XP009011567, ISSN: 1043-4666, DOI: 10.1016/1043-4666(92)90057-X
- Raymond R Brown ET AL: "Altered Tryptophan and Neopterin Metabolism in Cancer Patients Treated with Recombinant Inter leu kin 2'", J; Departments of Nutritional Sciences Madison, 1 September 1989 (1989-09-01), pages 4941-4944, XP055289658, Retrieved from the Internet: URL:http://cancerres.aacrjournals.org/cont ent/canres/49/17/4941.full.pdf [retrieved on 2016-07-19]

## Description

### FIELD OF THE INVENTION

The invention relates to *in vitro* diagnostic means for determination of kynurenine in body fluids and urine, and a medicament.

### BACKGROUND OF THE INVENTION

Contrast media (CM) are drugs for improving the visibility of internal organs and structures in X-ray based imaging techniques. They may have side effects ranging from itching to a life-threatening emergency described as contrast-induced nephropathy (CIN). The term CIN describes an acute renal failure which occurs within 24-72 hrs following administration of a contrast medium (CM) and for which there is no other explanation. CIN has been defined by the Contrast Media Safety Committee (CMSC) of the European Society of Urogenital Radiology (ESUR) as an increase in serum creatinine (SC) of at least 25 % or 44 µmοl/l within 3 days after CM administration in the absence of an alternative etiology (Fliser D et al., Nephrol. Dial. Transplant. 2012;27:4263-72*)* CIN occurs in up to 5% of hospitalized patients with normal renal function prior CM administration, but the prevalence increases to 20 to 30% in high risk patients (Golshahi J et al., J Nephropathol. 2014;3:51-6). Patients with a pre-existing renal impairment and diabetes are particularly vulnerable to a CIN. The true prevalence of CIN however is likely higher as patients can be asymptomatic. In a study including 7586 patients undergoing percutaneous coronary intervention 3.3% experienced acute renal failure (ARF) and 12.1% died within one year (44.6 % died within 5 years after). Thus, the incidence of death following a CIN was 0.4 % after one year and 1.5% after 5 years (Rihal CS et al., Circulation. 2002, 105(19):2259-64*).* This was 3.5-fold to patients without ARF which correlates with figures described previously (cf. McCullough et al., Am J Med 1997, 103(5):368-375; McCullough et al., JACC 2008, 51(15):1420-1428). An estimated 80 million parenteral doses of iodinated contrast media (CM) were given during 2003 and with number increasing there will more than 200 million parental doses of contrast media by year 2023 which by then would correspond to more than 1,000,000 CIN induced deaths within 1 to 5 years after intervention.

The mechanisms underlying CIN are not well understood. As they involve a reduction in medullary blood flow which leads to hypoxia and tubule cell damage, high-risk patients undergoing CM intervention therefore require a monitoring of renal function, e.g. by measuring serum creatinine and calculating the eGFR before and once daily for 5 days after the procedure. Nephrotoxic medications should further be discontinued, if possible, and the CM administered at lowest dosage. Oral hydration should be encouraged. High-risk patients may further receive intravenous hydration and a medication to ameliorate the effects of the contrast media (CM) such as N-acetylcysteine. Most patients display a partial or complete recovery of kidney function following a CIN but some suffer from a long-term loss of kidney function, cardiovascular events, and death (Pannu N et al., Clin J Am Soc Nephrol. 2013;8:194-202*;* Sigterman TA et al., Eur J Vasc Endovasc Surg. 2015*;* James MT, Wald R., Clin J Am Soc Nephrol. 2014;9:435-6*).*

There is no biomarker or diagnostic strategy which can predict CIN and CIN-related death. A volume-to-creatinine clearance ratio (v/CrCI) of 2.62 has been suggested as a safe limit but there is no "safe" dose of CM as limited doses of CM may cause CIN in high-risk patients (Laskey et al., JACC 2007, 50(7):584-590). Cystatin C has been described as a predictor of CIN but it is a biomarker for a *preexisting* renal impairment only but not for CIN and death (Wacker-Guβmann et al, AJR Am J Roentgenol. 2014, 202(2):452-8 - doi: 10.2214/AJR.13.1068; Kim et al., J Vasc Surg. 2015, pii: S0741-5214). Neutrophil gelatinase-associated lipocalin (NGAL) also increases after CM intervention and can not be used for pre-procedure prediction of CIN *(*Valette et al., Int Care Med 2013, 39:857-865). Studies testing KIM-1 are inconsistent (Shao X et al., PLoS ONE 2014,9: Article IDe84131). Homocysteine has further been studied as pre-procedure predictor for CIN *(*Li et al, Clin ChimActa. 2015 Feb 14;444:86 91. doi: 10.1016/j.cca.2015.02.019*)* The predictive value of homocysteine is however as weak as the one cystatin C. The best predictive value for CIN is provided by the cystatin C/creatinine-ratio while CIN in patients with creatinine within the upper normal range is significantly more frequent after intra-arterial than after IV contrast administration. These results represent therefore no improvement because physicians want to know prior administration of CM whether or not the patient is at risk to CIN and not whether or not the patient is suffering from renal impairment or other vascular problems. Holmes et al "Oxidative tryptophan metabolism in renal allograft recipients: increased kynurenine synthesis is associated with inflammation and OKT3 therapy" (Cytokine, Academic press Ltd, vol. 4, no. 3, 1 May 1992, p.205-213*)* disclose a method for determining the risk of renal graft rejection in renal allograft recipients by determination of the kinurenine levels in serum.

In essence, conventional biomarkers and assays cannot distinguish between the risk of CIN caused by an administration of CM and the risk of a major adverse renal event (MARE) which follows an existing renal impairment. Thus, there is an obvious need for an in vitro diagnostic for identifying patients at risk of CIN and in need of a medication for endothelial protection prior an administration of contrast media.

### SUMMARY OF THE INVENTION

This problem has been solved by an assay for testing whether a subject is predictive of CIN following CM invention and in need of a medication for endothelial protection, comprising taking a sample of body fluid from said subject and measuring the concentration of kynurenine in said sample and determining the need of an endothelial protective treatment for limiting the risk of CIN or AKI or AFR when the concentration of kynurenine in said sample is above a pre-determined threshold. The sample of body fluid may be serum, plasma, CSF, saliva or urine. Preferred embodiments of this method have been disclosed in the dependent claims.

A preferred embodiment relates to a method for in-vitro diagnosis of an increased risk for contrast-induced nephropathy (CIN) in a patient prior the patient being subjected to coronary angiography or an examination requiring an administration of contrast media, wherein the concentration of L-kynurenine in serum is determined in a serum sample of said patient and diagnosing an increased risk for death, dialysis treatment, a major adverse renal event (MARE) and/or a major adverse cardiovascular event (MACE) when the concentration of L-kynurenine (KYN) in serum is above a threshold value.

Another aspect of the disclosure relates to a method for determining the need of an endothelial protective treatment when a patient is subjected to coronary angiography or an examination requiring the administration of a contract medium, wherein the concentration of L-kynurenine is determined in a sample of body fluid of said patient taken prior to said treatment and diagnosing a need for an endothelial protective treatment when the concentration of L-kynurenine (KYN) is above a predetermined threshold value. The body fluid is preferably serum, EDTA plasma, urine or saliva.

A further aspect is a method of in-vitro diagnosis of subjects predictive of a major adverse renal event (MARE) and/or a major adverse cardiovascular event (MACE) or dialysis when subjected to coronary angiography or an examination involving the application of contrast media, comprising the steps of (i) providing a sample of serum or EDTA plasma from said subject; (ii) derivatizing quantitatively the L-kynurenine in said sample; (iii) determining the concentration of L-kynurenine by a competitive enzyme-linked immunoassay employing antibodies against derivatized L-kynurenine; and (iv) comparing the measured L-kynurenine concentration with a threshold value predictive for CIN, MARE, MACE, dialysis or death. In a preferred embodiment of said method the threshold value is 3,5 µmol kynurenine/L. Said sample of serum has been taken prior the application of any contrast media.

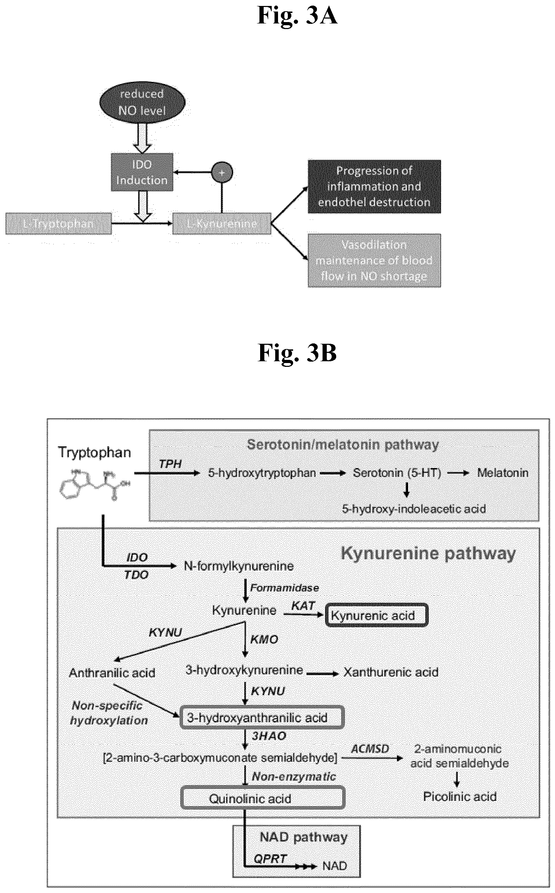

The present disclosure further contemplates a determination of the kynurenine to tryptophan ratio and/or a co-determination of the levels of creatinine and cystatin C for diagnosis whether or not said subject is suffering from an acute renal injury. It is further contemplated measuring the downstream products of the kynurenine pathway (see Fig. 3B), namely 3-hydroxy anthranilic acid (3-HAA), quinilinic acid (QuinA) and kynurenic acid (KynA) all known playing an important role in endothelia protection and/or the induction of adverse events. High 3-HAA levels indicate a state of protection of endothelia and decrease of cholesterin and lipides, and tryptophan metabolite 3-HAA further a lowering of plasma lipids, decreasing atherosclerosis. KynA stands for the instability of atherosclerotic plaques, a deteriorating status of atherosclerosis, which can lead to adverse events.

### BRIEF DESCRIPTION OF THE DRAWINGS

The present invention is best understood when read in conjunction with the accompanying figures, which serve to illustrate the preferred embodiments. It is understood, however, that the invention is not limited to the specific embodiments disclosed in the figures.
- **Fig. 1A**: **is** a diagram showing the odds rates for dialysis, MARE, and all case death between: Q1-Q3 : KYN < 3,5 µM, Q4 KYN > 3,5 µmol/l, by measuring KYN before CM administration (three-months follow-up);
- **Fig. 1B**: is a bar diagram showing the mean ratios of QuinA/KynA (downstream ratio of quinolinic acid to kynurenic acid) for MARE patients with pre-procedural KYN > 3,5 µmol/L and KYN < 3,5 µM prior CM intervention;

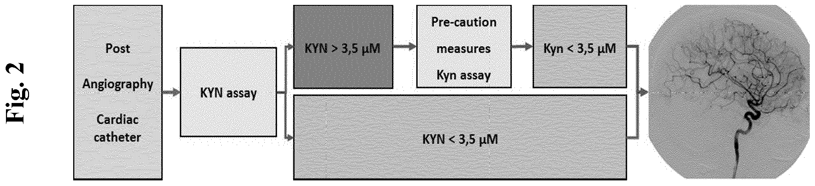

- **Fig. 2**: is a scheme for an in-vitro diagnosis of an increased risk for CIN, MARE or death or a need for endothelial protective treatment;
- **Fig. 3A**: is a scheme of presumed kynurenine action in endothelia wherein in case of an NO shortage IDO is active to produce kynurenine from tryptophan for vasodilation and for upholding blood microcirculation; when NO production cannot be restored high kynurenine levels appear to be a sign of enduring and progressing inflammation so that the endothelia is harmed until patient will suffer a major cardiovascular event (MACE) or major adverse renal event (MARE);
- **Fig. 3B**: is a diagram shown the serotonin/melatonin and the kynurenine pathways;
- **Fig. 4**: is a scheme of the procedure used in clinical study II for determining an increased risk for CIN, MARE or death following CM intervention;
- **Fig. 5**: is a diagram showing the distribution of baseline serum kynurenine concentrations of the cohort described in clinical study II.

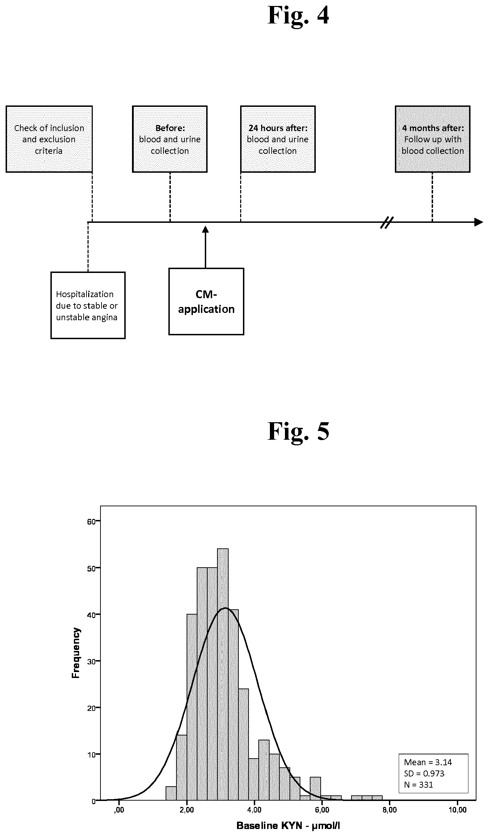

- **Fig. 5**: is a diagram showing the ROC graph (receiver operating characteristics) of baseline serum kynurenine concentrations as test variable and MARE until 4-months follow up as state variable.

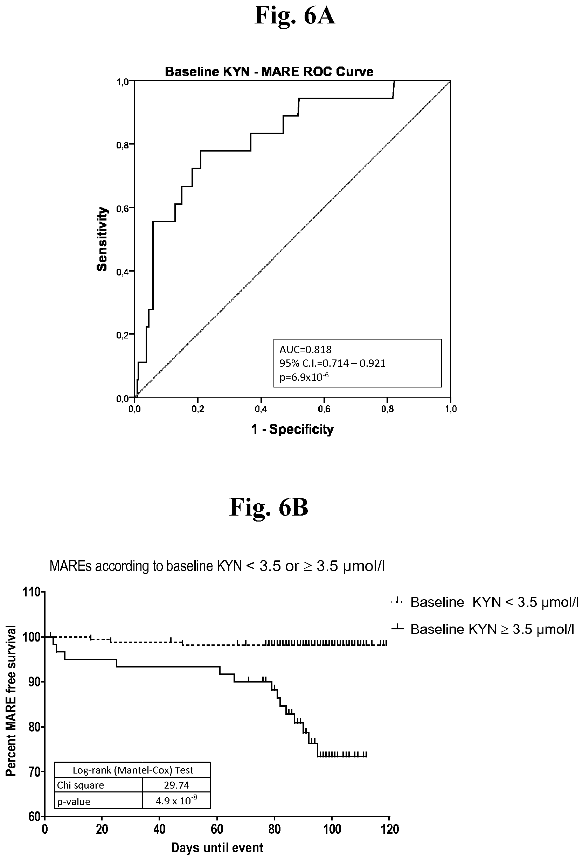

- **Fig. 6**: is a diagram of the Kaplan-Meier analysis of MARE until 4-months follow up in patients with baseline kynurenine concentrations < 3,5 µmol/L and > 3,5 µmol/L.

### DETAILED DESCRIPTION OF THE INVENTION

The present disclosure provides an in vitro method of identifying subjects prone of developing a contrast media induced nephropathy (CIN) prior any CM media application. This method allows medical intervention and treatment *prior* any severe renal insufficiency such as MARE and/or MACE. While increases in metabolites of the L-tryptophan-kynurenine pathway have been observed in serum and cerebrospinal fluid (CSF) in humans with renal insufficiency we have discovered that pre-procedural increased L-kynurenine levels can be observed in subjects predictive of MARE, MACE, dialysis or death when subjected to a CM intervention for coronary angiography or any other examination requiring the application of contrast media. Thus, the kynurenine level in body fluids is indicative for the endothelial status of a patient, say prior any acute inflammation or severe renal insufficiency can be observed. This is different to a disclosure testing for increased kynurenine levels in saliva for early detection of potential rejection problems in transplant patients (WO 2014/177680). Transplant patient have already received a immunologically problematic transplant or graft whereas the present disclosure relates to a pre-procedural examination, say an examination and testing prior any contrast-media intervention or administratiion.

Kynurenine is a metabolite of the essential amino acid L-tryptophan which is a precursor for various physiologically active biogenic amines. Tryptophan is metabolized in the human body in two main routes: the serotonin and the kynurenine pathways. The serotonin pathway is the better known, though more than 95% of the tryptophan goes the kynurenine route. The catabolism of L-tryptophan to L-kynurenine is the first and rate-limiting step of the kynurenine pathway and mediated by indoleamine-2,3-dioxygenase (IDO) and L-tryptophan-2,3-dioxygenase (TDO). The latter enzyme is predominantly located in liver cells and to a smaller degree in brain. IDO however is expressed in numerous cells (fibroblasts, macrophages, dendritic cells, etc.) throughout the human body while endothelial cells appear to be the primary site for IDO expression. The expression of IDO is induced by pro-inflammatory cytokines, in particular by interferon-y (IFN-y), interleukin-1 (IL-1) and to a lesser extent by tumor necrosis factor-α (TNF-α). Interleukin-1 (IL-1) and TNF-α have direct effects on the endothelial surface. Thus, the kynurenine pathway is significanty up-regulated with the immune response is up-regulated.

Without being bound by any theory, the inventor believes that the concentration of kynurenine in serum reflects the severity of inflammation and a need for immunomodulation and vasodilation. Literature suggests that an activated IDO induces homeostatic mechanisms against excessive immune reactions, promoting immune tolerance and eliciting anti-inflammatory effects. When pro-inflammatory cytokines are secreted into in the circulation following CM invention, tissue factor is expressed on the surfaces of the endothelium and of monocytes, which induces the production of the pro-inflammatory thrombin. IL-1 and TNF-α lead to the production of plasminogen activator inhibitor-1, a potent inhibitor of fibrinolysis. The pro-inflammatory cytokines are known to disrupt the body's naturally occurring modulators of coagulation and inflammation.

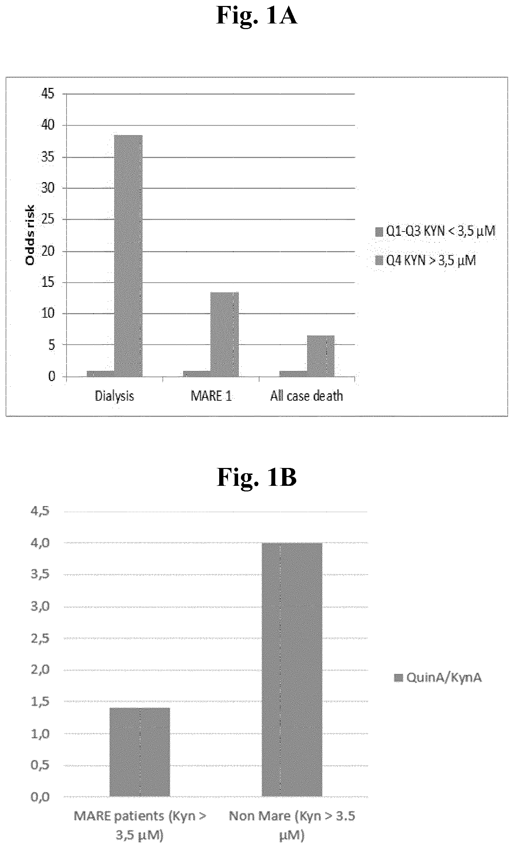

IDO is activated in various diseases, leading to decreasing tryptophan and increasing kynurenine blood levels. Moreover, chronic systemic low-grade inflammation enhances IDO activation. Chronic kidney disease (CKD) is accompanied by immune dysregulation and CKD patients display increased kynurenine plasma levels. Animal and cell culture experiments have further shown that kynurenine does not just accumulate in CKD patients due to impaired kidney function but that kynurenine plays possibly a role in the pathogenesis of CKD. Given the current evidence, kynurenine level in serum may not only serve as a new biomarker for assessing the risk of kidney disease and CIN following CM intervention but that it is represents a potential therapeutic target. The present inventors therefore contemplate a therapeutic intervention with the cascade of inflammation by controlling the knyurenine level in serum. In particular, drugs that finally lead to an increase in the ratio of QuinA/KynA and/or HAA/KynA may protect from MARE. MARE patients have a significantly lower QuinA/KynA ratio (mean value 1.4) than NON--MARE patients (mean value 4.0); see also Fig. 1B. Thus, a therapeutic intervention may result from drugs which increase the ratio of QuinA/KynA or HAA/KynA in the patients and which may therefore have a protective effect.

Such a protective effect could possibly be achieved by an administration of synthetic tryptophan analogs, endogenous tryptophan metabolites cinnabarinic acid, structural analogs (e.g. quinolinone-based ABR-215062 (Laquinimod)), pyrimidine synthesis inhibitors (Teriflunomid and Leflunomid) and N-(3,4-dimethoxycinnamoyl) anthranilic acid (3,4-DAA; Tranilast). For countering effects and side effects indoleamine-2,3-dioxygenase inhibitors (1MT and Berberine) and kynurenine-3-monooxygenase inhibitors (nicotinylalanine and Ro 61-8048) may be used. The kynurenine pathway is a promising target not only for affecting the immune system and for neuroprotection but also for obtaining endothelial and tubular protection through a control of pro-inflammatory mediators and cytokines. Drugs that further enhance the activity of IDO and TDO are acetylsalicylic acid (Aspirin), chlorpromazine, and reserpine. The catabolism of tryptophan to kynurenine is also up-regulated by inflammatory cytokines.

In this context, Tranilast has been shown to have a protective effects for endothelia in animal experiment (Cole JE et al. (2015), PNAS (2015) 112(42). The data from these experiments have demonstrated that the endogenous production of tryptophan metabolites through IDO is an essential feedback loop that controls atherogenesis and athero-inflammation. The IDO pathway further induces production of IL-10 in B-cells *in vivo* and immunoregulatory functions of B cells in atherosclerosis. The beneficial effects of anthranilic acid derivatives in atherosclerosis also suggest a novel therapy of CVD (Noakes, R. R. (2013), International Journal of Tryptophan Research : IJTR, 6, 67-71. doi:10.4137/IJTR.S12797).

3-HAA analoga such as Laquinimod, Teriflunomid and Leflunomid can modulate the kynurenine pathway and impact the QuinA/KynA and/or 3HAA/KynA ratios while the mode of action remains to be examined. Laquinimod has been tested in a clinical phase II study for therapy of Morbus Crohn (D'Haens G et al, Gut, 1-9. doi:10.1136/gutjnl-2014-307118). A similar amelioration of inflammation may be obtained for CDK, more precisely, by suppression of pro-inflammatory IL-10. Furthermore kynurenine aminotransferase inhibitors like cyclic hydroxamic acid PF-04859989, (**S**)-4-(ethylsulfonyl)benzoylalanine, BFF-122, BFF-816 may be used in therapeutic approaches while these drug have so far not be tested for conditions with an inflammatory background.

Kynurenine is a potent vasodilator which mediates its effects independently of nitric oxide. The kynurenine level in serum can therefore be used as a biomarker for the compensatory pathway regulating vascular function in inflammatory conditions. The ratio of kynurenine to tryptophan further describes the activity of IDO in serum. IDO is expressed throughout the human body but vascular endothelial cells are the primary site for IDO expression in systemic inflammatory conditions. IDO-induced conversion of tryptophan is therefore an important back-up system in conditions of reduced NO activity. IDO is a heme-containing enzyme and inhibited by NO. Conversely, metabolites of the kynurenine pathway have been shown to inhibit NO synthase. Thus, balanced activation of these two vasodilator systems is pivotal for maintenance of vascular function in inflammatory conditions.

In CVD patients, glomerular, tubular, and renal microvascular inflammatory changes are common without overt ischemic nephropathy. Histological examinations have revealed close similarities between glomerulosclerosis and atherosclerotic lesions, suggesting common pathogenesis. Kynurenine is produced locally in the kidneys upon stimulation by interferon gamma. Elevated kynurenine levels in serum may therefore reflect a renal induction of the IDO enzyme. The causes of CIN have not yet been elucidated. The side effects of contrast media range from a mild inconvenience to a life-threatening emergency. Medium severe forms of adverse reactions include allergic reactions, anaphylactic reactions, and cutaneous reactions. In general, a CM intervention appears to activate the immune system in a way difficult to control. Previous allergic reactions to contrast material generally increase the risk of adverse reactions. Pretreatment of such patients with a corticosteroid, hydrocortisone, methylprednisolone and/or diphenhydramine decreases the chance of allergic reactions. Awareness of risk factors and screening for their presence before CM application will allow early recognition of adverse reactions and their prompt treatment. Other major interacting parameters may be insufficient NO or prostaglandins. Besides its effects on the immune response, kynurenine has been identified a endothelium-derived vasodilator. Thus, balancing kynurenine levels in serum be pivotal for maintenance of vascular functions in inflammatory conditions.

The present application teaches that high kynurenine levels in serum prior CM administration, a pre-procedure to coronary angiography, represent an increased risk of death by a major adverse renal event (Death, odds risk per µmol/l KYN is 2.93 Q4 vs. Q1, p<0.001). Furthermore, high risk patients such as cardiac arrest patients are threatened (ICU death, odds risk per µmol/l KYN is 1.43; p<0.002). CIN is not common in patients with normal preexisting renal function; rather, it occurs more frequently in patients with renal impairment and is possibly exacerbated when the impairment is due to diabetic nephropathy. In brief, the pre-procedure kynurenine level in serum, prior CM administration, is predictive for major adverse renal events (MARE).

When radiographic contrast media are injected intravenously or intra-arterially, they pass from the vascular compartment through capillaries into the extracellular space. They are eliminated almost entirely by glomerular filtration, concentrated in the tubular lumen by water tubular reabsorption, thereby visualizing the urinary tract. The use of contrast media may lead to kidney dysfunction, especially in patients with preexisting renal impairment and in those with diabetes. Contrast-induced nephropathy (CIN) or contrast-induced acute kidney injury (CI-AKI) is an iatrogenic disease which has become a significant source of hospital morbidity and mortality. It is likely the third leading cause of hospital-acquired acute renal failure (after surgery and hypotension) accounting for 12% of all cases and it occurs in up to 5% of hospitalized patients who exhibit normal renal function prior CM intervention.

CIN as acute renal failure occurring within 24-72 hrs of exposure is commonly a nonoliguric and asymptomatic transient decline in renal function, generally occurring within 24 hrs. of CM administration, usually peaking on the third to fifth day, and returning to baseline within 10-14 days. Consequently, urinary levels of kynurenine or tryptophan are no reliable biomarkers for an imminent renal failure. The impairment of renal function is also mirrored by the absolute (0.5 mg/dL or greater) or relative (by 25% or greater) increase in serum creatinine from baseline. The increase or decrease in in serum creatinine levels after CM intervention is being interpreted as indicating nephrotoxicity even though such variation may occur even without contrast media administration. For this reason, the decrease of creatinine clearance has been discussed but measurement of creatinine clearance, as derived from 24-hour urine collection, is a cumbersome, impractical, and inaccurate test. The estimated glomerular filtration rate (eGFR) is more accurate and significantly easier to obtain as it is calculated from serum creatinine, age, gender, and ethnicity using the modification of diet in renal disease (MDRD) calculation or the very simple Cockcroft-Gault formula: (140 - number years of age) x Kg body weight/72/mg% of serum creatinine; in females the result x 0.85. Moderately decreased renal function is defined as eGFR 30-60 mL/min (renal insufficiency). In some cases, CIN may cause a more severe impairment of renal function with oliguria (<400mL/24hrs), requiring dialysis. In these cases, the mortality is much higher. Permanent severe renal failure requiring dialysis has been shown to occur in up to 10% of patients with preexisting renal failure who develop further reduction in renal function after coronary angiography or in <1% of all patients undergoing percutaneous coronary intervention using contrast agents. The management of CIN is the same as that for acute renal failure due to other causes. As will be apparent from the above the described method of predicting the risk of MARE or MACE or even death in case of a CM intervention represents a huge advantage vis-à-vis prior art diagnostic methods.

For outpatients, the risk for CIN, particularly in patients with creatinine clearance >45mL/min per 1.73 m , seems extremely low (approximately 2%). However, when an extraordinary risk for CIN is detected alternative methods to coronary angiography are recommended and/or preventive hydration (Awal et al, Mymensingh Med J. 2011 Apr;20(2):264-9; Stacul F et al, Contrast induced nephropathy: updated ESUR Contrast Media Safety Committee guidelines. European radiology. 2011, 21(12):2527-41. Epub 2011/08/26) and/or an administration of allopurinol or acetylcystein. The administration of statins have further been proposed. Prophylactic oral administration of allopurinol along with hydration may protect against CIN in high-risk patients undergoing coronary procedures (Erol et al, Int J Cardiol. 2013(20) 167(4):1396-9. doi: 10.1016/j.ijcard.2012.04.068; Chen SL et al, Int J Cardiol. 2008, 126(3):407-13). Thus, there are also conventional methods available of monitoring and lowering the risk of CIN when in the pre-procedure an in-vitro risk of MARE following CIN is detected, say by determining the concentration of kynurenine in serum of a patient who will be subjected to coronary angiography or a diagnostic method which requires the application of contrast media i.v. or i.a.

### EXAMPLES

### Example 1 - Measurement of L-kynurenine

L-Kynurenine was measured using a commercially available highly sensitive ELISA for competitive determination of L-kynurenine in human EDTA plasma, serum and urine (IDK Kynurenine ELISA - K 7728, Immundiagnostik AG, Bensheim, DE). This assay is based on the method of competitive enzyme linked immunoassays. In brief, the sample preparation included the addition of a non-stoichiometric amount (excess) of derivatization reagent in DMSO for L-kynurenine derivatization as described in EP 2,612,147 (Kellner et al) and US 4,818,683 A (Morel et al). The treated serum samples were then incubated with polyclonal anti-L-kynurenine-antibodies in wells of a microtiter plate coated with L-kynurenine-derivative (tracer). The target L-kynurenine in the sample then competed with the tracer, immobilized on the wall of the microtiter wells, for the binding of the polyclonal anti-L-kynurenine antibodies. As the L-kynurenine in the treated sample displaced the antibodies out of the binding to the tracer the concentration of tracer-bound antibody was inverse proportional to the L-kynurenine concentration in the treated sample.

In a second incubation step, a peroxidase-conjugated antibody was added to each microtiter well to detect bound anti-kynurenine antibodies. After washing-off of unbound components, tetramethylbenzidine (TMB) was added as a peroxidase substrate for a conventional color reaction. The enzymatic reaction was terminated by an acidic stop solution (2 M H₂SO₄). The color changes from blue to yellow and the absorbance was measured in the photometer at 450 nm. The intensity of the yellow color was then in this system inverse proportional to the L-kynurenine concentration in the sample. In other words, a high L-kynurenine concentration in the sample reduced the concentration of tracer-bound antibodies and lowered the photometric signal. A dose response curve of absorbance unit (optical density, OD at 450 nm) vs. concentration is generated using the values obtained from the standards so that the L-kynurenine present in the target samples could be determined directly from this curve.

More precisely, based on studies with samples of apparently healthy subjects a mean value of 2.22 µmol/l was obtained for EDTA plasma (n=43) and for serum (n=46). The standard deviation was 0.49 µmol/l respectively. Mean value ± 2x standard deviation: 2.2 ± 1.0 µmol/l. The normal kynurenine concentration in EDTA plasma and serum therefore ranges from 1.2 - 3.2 µmol/l

**TABLE 1**

| *Precision and reproducibility in serum* | | |
|---|---|---|
| Intra-assay (n=6) Sample | L-kynurenine [µmol/l] | CV [%] |
| 1 | 1.82 | 8.1 |
| 2 | 1.70 | 8.5 |

| Inter-assay (n=8) Sample | L-kynurenine [µmol/l] | CV [%] |
|---|---|---|
| 1 | 2.00 | 6.1 |
| 2 | 1.93 | 5.2 |

Two serum and two EDTA plasma samples were spiked with different L-kynurenine concentrations and measured in this competitive immunoassay. The mean recovery rate was 96.0 % for serum and 95.5% for EDTA plasma (n=2).

**TABLE 2**

| *Spiking recovery in serum* | | | |
|---|---|---|---|
| Spike [µmol/l] | L-kynurenine expected [µmol/l] | L-kynurenine measured [µmol/l] | Recovery [%] |
| sample 1 | | 1.98 | |
| 4.5 | 6.48 | 5.91 | 91.2 |
| 9.0 | 10.98 | 10.56 | 96.2 |
| sample 2 | | 2.13 | |
| 4.5 | 6.63 | 6.81 | 102.7 |
| 9.0 | 11.13 | 10.44 | 93.8 |

*Dilution recovery.* Two spiked serum and EDTA plasma samples were further diluted with reaction buffer and measured in this assay. The mean recovery rate was 103.8 % for serum, and 100.7 % for plasma (n=2).

*Analytical sensitivity.* The zero-standard was measured 48 times. The detection limit was set as Bo - 2 SD and estimated to be 0.04 µmol/l. Considering the dilution factor, the detection limit was calculated to be 0.12 µmol/l.

*Specificity.* Specificity was tested by measuring the cross-reactivity against compounds with *structural* similarity to L-kynurenine. The specificity is calculated in percent in relation to the L-kynurenine binding activity. 3-HK (3-hydroxy-DL-kynurenine) < 0.5 %; L-tryptophan < 0.2 %.

Consequently, the test as described herein proved useful for determining L-kynurenine in the relevant physiological concentrations and as pre-procedure predictor whether a patient is potentially at risk suffering from CIN, MARE, DIALYSIS or DEATH.

### Example 2 - Clinical Study I

An own funded study including 348 medium risk patients was conducted in 2014 for elucidating the role of kynurenine in relation to major adverse events in CIN. In addition to cystatin C and creatinine, L-kynurenine (KYN) was therefore measured in serum as described in example 1 prior and after CM application (Kim GS et al, J Vase Surg. 2015 Jan 13. pii: S0741-5214(14)02228-9. doi: 10.1016/j.jvs.2014.11.079.) The patients were followed up for 3 months from CM administration. Within this period 18 patients (3.3%) suffered from a major adverse renal event (MARE), 9 patients suffered from CIN leading to death and/or dialysis and 11 patients required dialysis treatment.

For further analysis and examination, we stratified the results in four quartiles (n=87), the first comprising patients with pre-interventional KYN-levels in serum of < 2.44 µmol/l (Q1), the second having 2.45-2.97 µmol/l (Q2), 2.98-3.49 µmol/l (Q3), and > 3.5 µmol/l (Q4). Within these quartiles we examined the odd rates (OR) to experience dialysis, MARE or death when comparing Q1-Q3 with Q4. The results of this analysis have been summarized in Fig. 1.

Surprisingly, odd rates (OR) in all groups were very high: (i) in dialysis: 38.50 [4.86-304.03] (N=12; p<0,001), (ii) in MARE: 13.40 [4.22-42.62] (N=18, p<0,001), and (iii) in all case death 6.52 [1.59-26.77] (N=9, p=0,003). The results for KYN have been in favor to the established markers creatinine (Cr) and cystatin C (Cys C) when measured prior CM administration (see Table 3)

**TABLE 3**

| *OR and correspondent signifiance for MARE 1 and all case deaths in Q1-Q3 and Q4 of the above mentioned cohort, for KYN, Cr and Cys C prior CM application* | | | |
|---|---|---|---|
| | KYN | Cr | Cystatin C |
| MARE 1 (n=18) | OR 38,4; p< 0,001 | OR 18,6 p< 0,001 | OR 17,5, p< 0,001 |
| All case deaths (n=9) | OR 13,4; p=0,003 | OR 9,4, p=0,059 | OR 6,4, p=0,067 |

KYN was in this study comparable to traditional markers Cr and Cystatin C when predicting MARE 1. KYN showed however significantly higher prediction compared to Cr and Cys C for all case death (p = 0,003 (vs. p = 0,059 in Cr, and p = 0,067 in Cys C). The results have been summarized below.

### Creatinine:

Dialysis: N=12; 18,644 [3,976-87,415] p<0,001
Death (all cases): N=9; 7,065 [1,717-29,060] p=0,002
Major cardiovascular event MACE (death): N=5; 5,077 [0,830-31,037] p=0,052
MARE1 (all cases, including death, dialysis, two-fold increase of serum creatinine after 3 months) : N=18; 9,445 [3,211-27,778] p<0,001.

### Cystatin c:

Dialysis: N=12; 17,500 [3,736-81,973] p<0,001
Death (all cases): N=9; 4,072 [1,063-15,604] p=0,028
MACE (death): N=5; 2,087 [0,342-12,747] p=0,416
MARE1 (all cases, including death, dialysis, 100% increase of serum creatinine after 3 months): N=18; 6,407 [2,296-17,885] p<0,001

### L-Kynurenine in serum

Dialysis: N=12; 38,50 [4,86-304,03] p<0,001
Death (all cases): N=9; 6,52 [1,59-26,77] p=0,003
MACE (death): N=5; 4,70 [0,769-28,68] p=0,066
MARE1 (all cases, including death, dialysis, 100% increase of serum creatinine after 3 months): N=18; 13,40 [4,22-42,62] p<0,001

Thus, the pre-procedure serum kynurenine levels proved to be an outstanding biomarker to predict CIN and MARE. Unlike other biomarkers pre-procedure kynurenine in serum can be easily determined prior CM application so that its measurement will allow a decision whether or not a patient is in need of endothelial protection. Different to serum cystatin C the serum kynurenine level is an independent predictor and most importantly, a pre-procedure biomarker of highly predictive value. Odds ratios for 1.-3. Q compared to Q 4: Q for creatinine (upper limit 1,39 mg/dl) and for cystatin C (1,33 mg/l) for endpoints dialysis, death, cardiovascular event (death) and "MARE1". Significance values (significant difference) after calculation of the Chi-Square value.

### Example 2 - Clinical study II

This prospective study was performed from January 2010 to December 2011 and approved by review board of the University Hospital Charité, Berlin, Germany. All clinical investigations were conducted according to the principles expressed in the Declaration of Helsinki. 331 patients with renal impairment (baseline creatinine > 1.1 mg/dL) or diabetes mellitus (diagnosed before study begin or HbA1c > 6.4%) undergoing coronary angiography were included. All patients without any present inclusion criteria or with terminal kidney disease were excluded. Additionally, patients who did not or were not able to provide their agreement to participate were excluded. The design of the study is illustrated in Figure 1. Data were analyzed using SPSS version 20.0 (SPSS, Inc, Chicago, IL, USA). Some of the figures were compiled with Graphpad Prism 5 (GraphPad Software Inc., La Jolla, California, USA). All values are given as mean ± standard deviation (SD). Unpaired t-test was used when comparing mean values of two groups. For the comparison of categorical variable distribution, chi square test was used. To assess specificity and sensitivity, receiver operating characteristic curves were calculated. Representing the best cutoff value, the point of the curve closest to the upper left corner of the coordinate system (sensitivity and specificity equal 1), was calculated and used to transform continuous parameters into a binary endpoint.

Patient treatment was not influenced in any case by this study. All patients received tri-iodonated non-ionic low-osmolar iobitridol (XENETIX® 350, Guerbet GmbH, Sulzbach/Taunus, Germany) as contrast medium. If patients were at high risk to develop CIN, the responsible physician administered 0.9% saline solution i.v. (500 ml before and two times 500 ml after CM examination). The mean age of the cohort was 68.8 ± 9.8 years, 23% (76) of all patients were female, 77% (255) were male, 98.8% (327) had a caucasian ethnic background, 1.2% (4) were non-caucasian and 16.1% (52) were current smokers. The average body mass index (BMI) was 29.0 ± 5.5 kg/m², mean HbA1c concentration was 6.8 ± 1.1 with 53.5% (177) of all patients being diabetics. Hypertension was present in 89.1% (294), 72.1% (238) of all patients suffered from coronary artery disease, 26.3% (87) had congestive heart disease and 27.5% (91) were anemic. In this study, after collection, pre-interventional blood samples were centrifuged for 5 min at 3000 rpm to obtain plasma. The pre-interventional plasma samples were then frozen and stored at -80 °C until further analysis.

The pre-interventional baseline serum KYN concentration was determined as described in Example 1 using specific antibodies against a kynurenine derivative (Article K7728 - Immundiagnostik AG, Bensheim, DE). The pre-interventional baseline serum KYN concentration mean turned out as 3.14 ± 0.97 µmol/L and was slightly increased compared with the mean baseline KYN concentration of apparently healthy subject. For the measurement of creatinine, the Jaffe method was used. Glomerular filtration rate was estimated by the Modification of Diet in Renal Disease (MDRD) formula. Cystatin c was measured by an immunonephelometric method using polystyrene particles coated with human cystatin c specific antibodies (Siemens Healthcare Diagnostics Products GmbH, Marburg, Germany). The descriptive statistics of the cohort prior CM administration have been summarized in Table 3 below:

**TABLE 3**

| *Descriptive statistics of the analyzed cohort* | |
|---|---|
| **Patient characteristics** | **All (n=331)** |
| Age, y | 68.8 ± 9.8 |
| Sex, female/male, % | 23.0/77.0 |
| Body mass index, kg/m2 | 29.0 ± 5.5 |
| Ethnicity (Caucasian, Non-Caucasian), % | 98.8/1.2 |
| Baseline kynurenine, µmol/L | 3.14 ± 0.97 |
| Baseline creatinine, mg/dL | 1.24 ± 0.45 |
| Baseline cystatin c, mg/L | 1.15 ± 0.46 |
| Baseline GFR, ml/min/1.73 m2 | 64.53 ± 21.44 |
| Diabetes mellitus, n, % | 177/53.5 |
| HbA1c, % | 6.8 ± 1.1 |
| Congestive heart failure, n, % | 87/26.3 |
| Coronary artery disease, n, % | 238/72.1 |
| Hypertension, n, % | 294/89.1 |
| Anemia, n, % | 91/27.5 |
| CM-volume, mL | 113.7 ± 57.0 |
| Saline hydration before/after CM, n, % | 178/53.9 |
| Smoking, n, % | 52/16.1 |

Patient subgroups with or without clinical events until follow-up, i.e. until four months after CM intervention, were then further statistically analyzed with respect measured pre-interventional serum KYN concentrations. In this statistical analysis, CIN has been defined as an increase of creatinine by 25% or 0.5 mg/dl from the baseline within 48 h or an increase of cystatin of 25% from the baseline within 24 h. Death is the subsumption of death of all causes. The endpoint dialysis has been defined as every dialysis in following four months after CM application. Non-elective hospitalization is every hospital stay for any reason, which was not preplanned at study entry.

The primary composite endpoint is the occurrence of a major adverse renal event (MARE) within the following four months (120 days) after CM application. MARE is defined as one of the following events: death, dialysis or a doubling of serum creatinine, as described above.

**TABLE 4**

| *Baseline serum Kynurenine concentrations (µmol*/*l) according to clinical events.* | | | | | | |
|---|---|---|---|---|---|---|
| Event | | Baseline KYN according to events | | | | |
| | | N | % | KYN | SD | P |
| CIN¹) | No | 276 | 83.4 | 3,147 | 0,968 | 0.822 |
| | Yes | 23 | 6.9 | 3,100 | 1,024 | |
| Death²) | Alive | 281 | 96.9 | 3,113 | 0,960 | 0.056 |
| | Dead | 9 | 3.1 | 4,208 | 1,466 | |
| Dialysis³) | No | 279 | 95.9 | 3,081 | 0,929 | <0.001 |
| | Yes | 12 | 4.1 | 4,816 | 1,118 | |
| Hospital⁴) | No | 240 | 83.3 | 3,093 | 0,967 | 0.050 |
| | Yes | 48 | 16.7 | 3,401 | 1,104 | |
| MARE⁵⁾ | No | 227 | 92.7 | 3,084 | 0,953 | <0.001 |
| | Yes | 18 | 7.3 | 4,400 | 1,213 | |

| | | | | | | |
|---|---|---|---|---|---|---|
| ¹⁾ *Elevation of serum creatinine by 25% or 0,5mg*/*dl from baseline in 48h.* ²⁾ *Death until follow up.* ³⁾ *Dialysis until follow up.* ⁴⁾ *at least one more non elective hospital stays until follow up.* ^{*5*)} *Death or Dialysis or doubling of serum creatinine until follow up.* | | | | | | |

The distribution of measured pre-procedural serum KYN concentrations is shown in Figure 1. Baseline creatinine concentrations were 1.24 ± 0.45 mg/dL and baseline cystatin c concentrations were 1.15 ± 0.46 mg/L. The average GFR was 64.53 ± 21.44ml/min/1.73 m2. Mean administered CM volume was 113.7 ± 57.0 mL and 53.9% (178) of patients received saline hydration before or after CM intervention.

CIN, death or hospitalization until follow up could not be associated with pre-interventional serum kynurenine concentrations. However, patients who received dialysis or developed MARE until the four months follow up displayed significantly elevated pre-interventional serum kynurenine levels. The predictive value of pre-interventional serum KYNURENINE level with respect to the primary endpoint of this study, MARE, was calculated by the area under the receiver operating characteristic graph (ROC-AUC), which is shown in Fig. 5.

The pre-interventional baseline serum KYN concentration prior CM application proved very accurate in predicting MARE (AUC=0.818; 95% C.I.= 0.714-0.921; p=6.9 x 10-6). The optimal cut-off value was < 3.5 µmol/L. The results have been summarized in Table 5 below which comprises the descriptive statistics of the studied cohort. Patients have been grouped therein with respect to baseline serum KYN concentrations < 3.5 µmol/L and ≥ 3.5 µmol/L prior CM application.

**TABLE 5**

| *Descriptive statistics of patients grouped according to baseline kynurenine concentrations < 3.5 µmol*/*L and > 3.5 µmol*/*L.* | | | |
|---|---|---|---|
| **Patient characteristics** | **KYN < 3.5µmol/L (n=248)** | **KYN ≥ 3.5µmol/L (n=83)** | **p-value** |
| Age, y | 68.0 ± 9.6 | 71.5± 9.8 | 0.004 |
| Sex, female/male, % | 21.8/78.2 | 26.5/73.5 | 0.375 |
| Body mass index, kg/m2 | 29.0 ± 5.5 | 29.1 ± 5.6 | 0.893 |
| Ethnicity (Caucasian, Non-Caucasian), % | 98.8/1.2 | 98.8/1.2 | 0.801 |
| CM-volume, MI | 113.5 ± 56.0 | 114.5 ± 60.1 | 0.889 |
| Baseline kynurenine, µmol/L | 2.69 ± 0.45 | 4.45 ± 0.92 | <0.0001 |
| Baseline creatinine, mg/dL | 1.13 ± 0.27 | 1.59 ± 0.64 | <0.0001 |
| Baseline cystatin c, mg/L | 1.01 ± 0.33 | 1.55 ± 0.55 | <0.0001 |
| Baseline GFR, ml/min/1.73 m2 | 69.86 ± 20.28 | 48.74± 16.47 | <0.0001 |
| Diabetes mellitus, n, % | 139/56.3 | 38/45.8 | 0.097 |
| HbA1c, % | 6.9 ± 1.1 | 6.5 ± 0.9 | 0.104 |
| Congestive heart failure, n, % | 53/21.4 | 34/41.0 | 0.0004 |
| Coronary artery disease, n, % | 180/72.9 | 58/69.9 | 0.599 |
| Hypertension, n, % | 214/86.6 | 80/96.4 | 0.014 |
| Anemia, n, % | 53/21.4 | 38/48.5 | <0.0001 |
| Saline hydration before/after CM, n, % | 118/47.6 | 60/73.2 | <0.0001 |
| Smoking, n, % | 43/17.8 | 9/11.1 | 0.158 |

Significant differences in patients with serum kynurenine concentrations **≥** 3.5 µmol/L were a higher age (p=0.004), higher baseline concentrations of creatinine and cystatin c (both p<0.0001), a lower GFR (p<0.0001), a higher prevalence for congestive heart failure (p=0.0004), hypertension (p=0.014) and anemia (p<0.0001). Additionally, these patients more often received saline hydration before/after CM interventions (p<0.0001).

The occurrence of MARE until follow up has further been looked at and patient characteristics according to this classifier have been summarized in TABLE 6.

**TABLE 6**

| *Descriptive statistics of patients grouped according to the occurrence of MARE until follow up.* | | | |
|---|---|---|---|
| **Patient characteristics** | **MARE until Follow Up (YES/NO)** | | |
| | **NO (n=227)** | **YES (n=18)** | **p-value** |
| Age, y | 68.0 ± 9.6 | 69.7 ± 11.7 | 0.648 |
| Sex, female/male, % | 29.1 ± 5.5 | 27.3 ± 5.0 | 0.950 |
| Body mass index, kg/m2 | 29.0 ± 5.5 | 29.1 ± 5.6 | 0.185 |
| Ethnicity (Caucasian, Non-Caucasian), % | 99.1/0.9 | 94.4/5.6 | 0.083 |
| CM-volume, mL | 115.8 ± 55.8 | 108.8 ± 71.4 | 0.619 |
| Baseline kynurenine, µmol/L | 3.08 ± 0.95 | 4.40 ±1.21 | <0.0001 |
| Baseline creatinine, mg/dL | 1.21 ± 0.35 | 2.02 ± 1.04 | 0.004 |
| Baseline cystatin c, mg/L | 1.12 ± 0.42 | 1.82 ± 0.78 | 0.001 |
| Baseline eGFR, ml/min/1.73 m2 | 65.5 ± 20.06 | 45.5 ± 26.5 | 0.0001 |
| Diabetes mellitus, n, % | 120/52.9 | 8/44.4 | 0.491 |
| HbA1c, % | 6.7 ± 1.1 | 6.2 ± 0.8 | 0.304 |
| Congestive heart failure, n, % | 57/25.1 | 10/55.6 | 0.005 |
| Coronary artery disease, n, % | 171/75.7 | 12/66.7 | 0.396 |
| Hypertension, n, % | 199/88.1 | 18/100.0 | 0.120 |
| Anemia, n, % | 57/25.1 | 10/55.6 | 0.005 |
| Saline hydration before/after CM, n, % | 125/55.1 | 10/55.6 | 0.968 |
| Smoking, n, % | 34/15.2 | 6/33.3 | 0.046 |

The most prominent significant difference between patients and patients with MARE until follow up was for baseline serum kynurenine concentrations (p<0.0001) prior CM application, followed by baseline eGFR, cystatin c and creatinine. Patients with MARE within 4 months following CM application had in general a higher prevalence for congestive heart failure and anemia and were more often smokers.

### Example 3 - Statistical analyses

Data were analyzed using SPSS version 20.0 (SPSS, Inc, Chicago, IL, USA). In order to analyze group specific occurrence of events over time we calculated the respective Kaplan-Meier curves and tested for statistical significant differences using the Log-rank Mantel-Cox test. To adjust for possible confounding, multivariate Cox regression analysis was performed. Probability values <0.05 were considered significant. The results have been summarized in TABLE 7 below.

**TABLE 7**

| *Multivariate Cox regression analyses.* | | | | | | |
|---|---|---|---|---|---|---|
| **Model A** Chi-square=58.074 p<0.0001 | **B** | **S.E.** | **Sig.** | **Exp(B)** | 95% C.l.for EXP(B) | |
| | | | | | Lower | Upper |
| Baseline KYN, µmol/L | 0.519 | 0.215 | 0.016 | 1.680 | 1.103 | 2.559 |
| Baseline eGFR, ml/min/1.73 m2 | -0.044 | 0.018 | 0.015 | 0.957 | 0.924 | 0.992 |
| Age, years | -0.040 | 0.025 | 0.116 | 0.961 | 0.915 | 1.010 |
| Sex (male, female) | -0.302 | 0.671 | 0.652 | 0.739 | 0.198 | 2.754 |
| Diabetes (yes, no) | 0.056 | 0.590 | 0.924 | 1.058 | 0.333 | 3.364 |
| Congestive heart failure (yes, no) | -0.866 | 0.535 | 0.106 | 0.421 | 0.148 | 1.200 |
| Anemia (yes, no) | -0.837 | 0.583 | 0.151 | 0.433 | 0.138 | 1.356 |
| Contrast media volume (mL) | -0.002 | 0.005 | 0.665 | 0.998 | 0.989 | 1.007 |
| Saline hydration before/after CM | 1.286 | 0.583 | 0.027 | 3.618 | 1.155 | 11.339 |

| **Model B Chi-square=50.373 p<0.0001** | **B** | **S.E.** | **Sig.** | **Exp(B)** | **95% C.I.for EXP(B)** | |
|---|---|---|---|---|---|---|
| | | | | | Lower | Upper |
| Baseline KYN | 2.067 | 0.710 | 0.004 | 7.901 | 1.965 | 31.767 |
| Baseline GFR, ml/min/1.73 m2 | -0.037 | 0.018 | 0.039 | 0.963 | 0.930 | 0.998 |
| Age | -0.038 | 0.026 | 0.142 | 0.963 | 0.915 | 1.013 |
| Sex (male, female) | -0.305 | 0.653 | 0.641 | 0.737 | 0.205 | 2.653 |
| Diabetes (yes, no) | 0.259 | 0.630 | 0.681 | 1.296 | 0.377 | 4.455 |
| Congestive heart failure (yes, no) | -0.927 | 0.548 | 0.091 | 0.396 | 0.135 | 1.158 |
| Anemia (yes, no) | -0.817 | 0.588 | 0.164 | 0.442 | 0.140 | 1.397 |
| Contrast media volume (mL) | -0.001 | 0.005 | 0.865 | 0.999 | 0.990 | 1.008 |
| Saline hydration before/after CM | 1.214 | 0.553 | 0.028 | 3.367 | 1.139 | 9.953 |

Model A used serum kynurenine as a continuous variable, model B used serum kynurenine categorical variable stratified into serum kynurenine < 3.5 µmol/L and **≥** 3.5 µmol/L. Covariates used in the models were baseline GFR, age, sex, the amount of administered contrast media, saline hydration before/after CM intervention and the presence of diabetes, congestive heart failure and anemia. Even after adjusting for these covariates, baseline serum kynurenine was independently associated with MARE (exp(B)= 1.680; 95% C.I.=1.103 -2.559; p=0.016;). Using serum kynurenine categorized into < 3.5 µmol/L and ≥ 3.5 µmol/L showed an even stronger independent association with MARE until follow up (exp(B)= 7.901; 95% C.I.=1.965 - 31.767; p=0.004;). Thus, baseline serum kynurenine ≥ 3.5 µmol/L was significantly associated with a higher prevalence of MARE until follow up (Log-rank Mantel-Cox test; Chi-square=29.74; p=4.9 x 10-8). The results have further been summarized in Figure 6. Consequently, the multivariate Cox-Regression models using established risk factors for MARE also demonstrate an independent association between baseline serum KYN **≥** 3.5 and an elevated risk for MARE.

### Summary of results

Measurement of pre-interventional serum kynurenine concentration (baseline KYN) prior CM application, e.g. for coronary angiography, is a predictive biomarker for the occurrence of major adverse renal events (MARE: death, need for dialysis, doubling of serum creatinine) up to four months after contrast media exposure due to coronary angiography in a population with moderate risk for CIN. The calculated threshold for kynurenine using the KYN-ROC graph for MARE resulted in a cut-off concentration for pre-interventional serum KYN so that patients predictive for MARE after coronary angiography can be easily identified. This is clinically very important information for patient's management. Patients predictive for MARE based on baseline kynurenine concentrations before intervention are in need of a much closer follow-up as compared to patients with low MARE risk. Thus, baseline serum kynurenine concentration are a tool for personalized patient care management after coronary angiography.

The current CIN definitions are based on short-term changes of glomerular function after CM application and use changes of surrogate biomarkers such as creatinine or cystatin C. These definitions ignore alterations in tubular function although the morphological hallmark of acute renal failure in general and CIN in particular are tubular alterations such as tubular necrosis and tubular dilatation. In other words, the currently used clinical definitions describe more likely short term alterations of glomerular hemodynamics / glomerular function which are not related to long-term outcomes whereas tubular alterations are closer linked to long term clinical consequences induced by contrast media (Chaykovska et al., PlosOne I DOI:10.1371/journal.pone.0145723 January 11, 2016) . As pre-interventional baseline kynurenine concentrations prior CM application are predictive for MARE, this biomarker likely reflects not the degree of tubular damage but is descriptive for endothelial function. Consequently, the present inventors contemplate also a predictive value for other indications such as reperfusion injury and reperfusion injury in AKI as well as immunology. With respect to chronic kidney disease (CKD), high serum kynurenine concentrations may indicate a fast progressing CKD.

The present invention also provides means for a pharmacological alteration of serum kynurenine concentration prior to coronary angiography which have an impact on the risk of MARE. In other words, serum kynurenine is not just a biomarker predictive for MARE but has endothelial and immunological effects which are closely related to the tryptophan-kynurenine ratio and IDO activity. Metabolism of tryptophan to kynurenine by indoleamine 2,3-dioxygenase (IDO) expressed in endothelial cells contributes to arterial vessel relaxation and control of blood pressure. Pharmacological inhibition of IDO increases blood pressure whereas tryptophan dilates arteries when active IDO and an intact endothelium were both present (Wang Y et al., Nature Medicine 16 (3): 279-85). It has been shown that kynurenine dose-dependently decreases blood pressure in hypertensive rats, inhibited contraction of arteries, and relaxed pre-constricted rings endothelium-independently. Arterial relaxation by kynurenine was mediated by activation of the adenylate and soluble guanylate cyclase pathways. Kynurenine is a potent endothelium-derived vasodilator.

Previous studies have demonstrated that metabolites of the kynurenine pathway are elevated in CKD animal models as well as in CKD patients (Pawlak D et al, . J. Physiol. Pharmacol. 2001;52:755-66; Pawlak D et al., Int Urol Nephrol. 2001;33:399-404; Pawlak D et al, J. Physiol. Pharmacol. 2003;54:175-89) This raises the question whether an impaired kidney function merely leads to elevated kynurenine levels, or whether elevated kynurenine levels are causally involved in the pathogenesis of kidney disease which would allow pharmacological intervention. The present invention submit the latter because kynurenine formamidase knock out animals display a significant increase of metabolites of the kynurenine pathway concomitant to a gradual deterioration of kidney function (Dobrovolsky VN et al, Biochimica et Biophysica Acta (BBA) - General Subjects. 2005;1724:163-72*.)* Furthermore it was demonstrated in mesangial cell culture experiments, that kynurenine stimulates pro-fibrotic gene expression (Yoshimura H et al, Exp. Mol. Pathol. 2009;87:70-5*)* Such observations indicate a possible pathological role of accumulated kynurenine metabolites in the progression of renal failure and might also serve as one possible explanation between the association of elevated serum kynurenine and an increased risk of MARE in this study.

The instant results show, that regardless of the underlying mechanism, baseline serum kynurenine concentration is a highly predictive biomarker for MARE up until 4 months after coronary angiography. Yet kynurenine was not associated with the short term renal outcome CIN and CIN itself was not associated with MARE. Current diagnostic classifications like ESUR defines CIN as an increase in serum creatinine (SC) of at least 25 % within 3 days after CM administration. CIN and other acute renal impairments thus are likely be under-diagnosed as serum creatinine displays a delayed rise in response to an acute renal failure triggering insult and lacks specificity due to extrarenal influences on serum concentrations. Furthermore, serum creatinine is insensitive to mild tubular injury. It is possible that not all patients who suffered from tubular damage following CM administration were categorized as CIN patients in this study, thus influencing the association between CIN and MARE. Recent reviews of clinical studies investigating AKI proposed that employing a renal composite endpoint like MARE can better define patients with a meaningful poor outcome. Moreover, this could overcome the limitation of competing risk factors associated with a single outcome (for example if a patient dies, the development of CKD is impossible).

## Claims

1. Method of *in-vitro* diagnosis of an increased risk for contrast-induced nephropathy (CIN) in a patient prior the patient being subjected to coronary angiography or an examination requiring the administration of a contrast medium, **characterized in that** the concentration of L-kynurenine in serum is determined in a serum sample of that patient and diagnosing an increased risk for death, dialysis treatment, a major adverse renal event (MARE) and/or a major adverse cardiovascular event (MACE) when the concentration of L-kynurenine (KYN) in serum is above a threshold value.

2. Method of in-vitro diagnosis patients in need of endothelial protective treatment prior the patient being subjected to coronary angiography or an examination requiring the administration of a contrast medium, **characterized in that** the concentration of L-kynurenine is determined in a sample of body fluid of that patient and diagnosing a need for an endothelial protective treatment when the concentration of L-kynurenine (KYN) in said sample is above a threshold value.

3. Method of in-vitro diagnosis of subjects predictive of a major adverse renal event (MARE) and/or a major adverse cardiovascular event (MACE) or dialysis prior coronary angiography or an examination involving the application of contrast media, **characterized by** the steps of:
providing a sample of serum or EDTA plasma from said subject;
derivatizing quantitatively the L-kynurenine in said sample;
determining the concentration of L-kynurenine by a competitive enzyme-linked immunoassay employing antibodies against derivatized L-kynurenine; and comparing the measured L-kynurenine concentration with a threshold value predictive for CIN, MARE, MACE, dialysis or death.

4. Method as claimed in claim 3, wherein the threshold value is 3,5 µmol kynurenine/L.

5. Method as claimed in any preceding claim 1 to 4, wherein the sample of serum is taken prior and after the application of any contrast media.

6. Method as claimed in any preceding claim 1 to 5, further comprising a determination of the kynurenine:tryptophan ratio levels.

7. Method as claimed in any preceding claim 1 to 6, further comprising a co-determination of the concentration of creatinine and cystatin C.

8. Method as claimed in any preceding claim 1 to 7, further comprising a determination of the ratio of quinilinic acid (QuinA) to kynurenic acid (KynA) and/or 3-hydroxy anthranilic acid (3-HAA) to kynurenic acid (KynA).

## Patentansprüche

1. Verfahren zur Feststellung einer erhöhten Gefahr einer Kontrastmittel-induzierten Nephropathie (CIN) *in-vitro* in Patienten vor deren Untersuchung mittels koronarer Angiographie oder einer Untersuchung, welche die Verabreichung von Kontrastmittel verlangt, **dadurch gekennzeichnet, dass** in einer Serumprobe des Patienten die Serum-Konzentration des L-Kynurenins bestimmt wird und man ein erhöhtes Mortalitätsrisiko diagnostiziert, eine Dialysebehandlung, ein MARE (Major Adverse Renal Event) und/ oder ein MACE (Major Adverse Cardiovascular Event), wenn die Serum-Konzentration des L-Kynurenins (KYN) oberhalb eines Schwellenwerts liegt.

2. Verfahren zur in-vitro Feststellung der Patienten, die eine endotheliale Schutzbehandlung benötigen bevor sie einer koronaren Angiographie unterworfen werden oder einer Untersuchung, welche die Verabreichung eines Kontrastmittels beinhaltet, **dadurch gekennzeichnet, dass** in einer Probe mit Körperflüssigkeit des Patienten die Konzentration von L-Kynurenin bestimmt wird und man den Bedarf einer endothelialen Schutzbehandlung feststellt, wenn die L-Kynurenin-Konzentration in der Probe oberhalb eines Schwellenwerts liegt.

3. Verfahren zur in-vitro Feststellung von Personen, die ein MARE (major adverse renal event) und/ oder ein MACE (Major adverse cardiovascular event) erleiden werden oder eine Dialysebehandlung benötigen werden und zwar vor einer koronaren Angiographie oder einer Untersuchung, welche die Verabreichung von Kontrastmittel verlangt, **gekennzeichnet durch** die Schritte
Bereitstellen einer Serumprobe oder einer Probe mit EDTA-Plasma der Person; quantitatives Derivatisieren des L-Kynurenins in der Probe;
Bestimmen der Konzentration des L-Kynurenins in der Probe durch einen kompetitiven Enzymgekoppelten Immunassay mit Hilfe von Antikörpern gegen das derivatisierte L-Kynurenin und Vergleichen der gemessenen L-Kynurenin-Konzentration mit einem Schwellenwert, der steht für CIN, MARE, MACE, Dialyse oder Tod.

4. Verfahren nach Anspruch 3, wobei der Schwellenwert ist 3,5 µmol Kynurenin/L.

5. Verfahren nach einem der vorstehenden Ansprüche 1 bis 4, wobei die Serumprobe vor und nach der Anwendung eines Kontrastmittels genommen wird.

6. Verfahren nach einem der vorstehenden Ansprüche 1 bis 5, zudem umfassend ein Bestimmen des Verhältnisses der Kynurenin:Tryptophan-Spiegel.

7. Verfahren nach einem der vorstehenden Ansprüche 1 bis 6, zudem umfassend ein Mitbestimmen der Konzentration von Kreatinin und Cystatin C.

8. Verfahren nach einem der vorstehenden Ansprüche 1 bis 7, zudem umfassend ein Bestimmen des Verhältnisses von Quinulinsäure (QuinA) zu Kynureinsäure (KynA) und/oder von 3-Hydroxy-Anthranilsäure (3-HAA) zu Kynureninsäure (KynA).

## Revendications

1. Procédé de diagnostic *in vitro* d'un risque accru de néphropathie induite par les produits de contraste (NIPC) chez un patient avant que le patient soit soumis à une angiographie coronaire ou à un examen requérant l'administration d'un milieu de contraste, **caractérisé en ce que** la concentration en L-kynurénine dans le sérum est déterminée dans un échantillon de sérum de ce patient et diagnostiquant un risque de mort accru, un traitement de dialyse, un événement rénal indésirable majeur (MARE) et/ou un événement cardiovasculaire indésirable majeur (ECIM) lorsque la concentration en L-kynurénine (KYN) dans le sérum est au-dessus d'une valeur seuil.

2. Procédé de diagnostic *in vitro* de patients en besoin d'un traitement protecteur endothélial avant que le patient soit soumis à une angiographie coronaire ou à un examen requérant l'administration d'un milieu de contraste, **caractérisé en ce que** la concentration en L-kynurénine est déterminée dans un échantillon de fluide corporel de ce patient et diagnostiquant un besoin concernant un traitement protecteur endothélial lorsque la concentration en L-kynurénine (KYN) dans ledit échantillon est au-dessus d'une valeur seuil.

3. Procédé de diagnostic *in vitro* de sujets prédictifs d'un événement rénal indésirable majeur (MARE) et/ou d'un événement cardiovasculaire indésirable majeur (ECIM) ou d'une dialyse avant angiographie coronaire ou un examen impliquant l'application de milieux de contraste, **caractérisé par** les étapes de :
fourniture d'un échantillon de sérum ou de plasma EDTA provenant dudit sujet ;
dérivatisation quantitative de la L-kynurénine dans ledit échantillon ;
détermination de la concentration en L-kynurénine par un dosage immuno-enzymatique par compétition employant des anticorps contre la L-kynurénine dérivatisée ; et comparaison de la concentration en L-kynurénine mesurée avec une valeur seuil prédictive de NIPC, MARE, ECIM, dialyse ou mort.

4. Procédé selon la revendication 3, dans lequel la valeur seuil est de 3,5 µmoles de kynurénine/L.

5. Procédé selon l'une quelconque des revendications 1 à 4, dans lequel l'échantillon de sérum est prélevé avant et après l'application de tout milieu de contraste.

6. Procédé selon l'une quelconque des revendications 1 à 5, comprenant en outre une détermination des niveaux de rapport kynurénine:tryptophane.

7. Procédé selon l'une quelconque des revendications 1 à 6, comprenant en outre une co-détermination de la concentration en créatinine et en cystatine C.

8. Procédé selon l'une quelconque des revendications 1 à 7, comprenant en outre une détermination du rapport acide quinilinique (QuinA) sur acide kynurénique (KynA) et/ou d'acide 3-hydroxy anthranilique (3-HAA) sur acide kynurénique (KynA).
